# EUROPEAN PATENT APPLICATION

(11) **EP 1 323 833 A1**
(43) Date of publication of application: **02.07.2003**
(21) Application number: 01963416.1
(22) Date of filing: 05.09.2001
(51) Int. Cl.: C12Q 1/68, C12N 15/09

(54) **METHOD OF DETERMINING BASE SEQUENCE**

(30) Priority: 08.09.2000 JP 2000272489
(71) Applicant: Ajinomoto Co., Inc., Tokyo 104-8315 (JP)
(72) Inventor: KIMURA, Eiichiro, Kawasaki-shi, Kanagawa 21 (JP)
(74) Representative: Strehl Schübel-Hopf & Partner
(86) International application number: JP0107671
(87) International publication number: WO02020839

(57) **Abstract**

A nucleotide sequence of a single nucleotide or plural nucleotides can be determined by assaying reactions in one reaction solution.

By detecting a reaction product continuously generated in an extension reaction system following the extension reaction of polynucleotide chain and thereby determining the alignment in the sequentially extending polynucleotide, the nucleotide sequence can be determined.

## Description

### Technical Field

The present invention relates to a method for nucleotide sequencing.

### Background of the Invention

Owing to the recent improvement of the nucleotide sequencing technology, a new research region of genome analysis has been developed, so that an enormous volume of nucleotide sequence data has been accumulated, which is represented by the determination of the overall human nucleotide sequence in practical sense. Furthermore, tools such as software for the analysis of such enormous volume of gene information have been developed. From now on, additionally, the new research region will have increased importance in the development of pharmaceutical products and the industrialization of gene therapy and gene diagnosis. In such circumstances, expectantly, the nucleotide sequencing technology will achieve a far greater efficiency at lower cost.

The methods currently used most widely for nucleotide sequencing adopt the fundamental principles and methods introduced by Sanger, F., Nicklen, S., and Coulson, A.R.[(1977) Proc. Natl. Acad. Sci. USA 74, pp. 5463-5467] and Maxam A.M. and Gilbert, W. [(1977) Proc. Natl. Acad. Sci. USA 74, pp. 560-564].

The Sanger's dideoxy sequencing method is widely used currently. Even to date, the Sanger's method is an excellent method for nucleotide sequencing based on single-stranded DNA template.

The method is described as follows by separating the reactions at individual steps. During polymerase extension reaction with a template DNA of which the sequence is intended to be determined, so as to elongate enzymatically the chain with four nucleotides, namely adenine (A), cytosine (C), guanine (G) and thymine (T), a dideoxynucleotide corresponding to each of the four nucleotides is randomly inserted in the position of the corresponding deoxynucleotide. Then, the resulting composite mixture thus obtained is subsequently separated by electrophoresis on polyacrylamide gel or using a capillary column packed with a gel, to determine the nucleotide sequence.

According to the method, as described above, a procedure for molecular fractionation after dideoxynucleotide incorporation is inevitable. The step complicates a series of nucleotide sequencing procedures and requires a large assay system.

Since then, various modifications have been made including the modification of the method of electrophoresis and the four types of deoxyribonucleotides and dideoxyribonucleotides as well as the use of fluorescent substances for detection. The relevant references are described below.
G.M.Church and S.Kieffer-Higgins, Science, 240, pp. 185-188 (1988)
International Publication WO 93/02212
Venter, C.J. et al., T.I.B.S. 10, pp.8-11 (1992)
Prober, J.M. et al., Science 238, pp. 336-341 (1987)
Mathies, R.A. and Huang, X.C., Nature 359, pp. 167-169 (1992)

As a method for identifying a nucleotide existing at a specific position in nucleic acid, the International Publication WO 93/02212 describes such method for identifying the nucleotide via dideoxynucleotide incorporation. In view of the requirement of electrophoresis, however, the method is identical ,to the aforementioned method. According to the method, further, point mutation can be detected but continuous nucleotide sequencing is impossible. According to the method, further, a primer should be arranged adjacent to the nucleotide, so it is impossible to characterize complicated mutations such as small insertion or deletion.

Alternatively, a method with no use of electrophoresis is also proposed and used practically.

Nucleotide sequencing by hybridization [Strezoska, Z. et al., (1991) Proc. Natl. Acad. Sci. USA 88, pp. 10089-10093] and tunnel effect microscopy [Driscoll, R. J. et al (1990) Nature 346, pp. 294-296] never require any electrophoresis step on gel. The technique using hybridization has been developed and under way of practical application, owing to the progress in the DNA chip technology. In this case, however, DNA for use in the hybridization should preliminarily be prepared by PCR and the like. As long as the hybridization method is used, further, a problem of mismatch hybridization to a similar sequence except for an intended sequence unavoidably exists. Further, the method is not appropriate for the determination of a novel sequence spanning plural nucleotides, although the method can detect point mutation. Still further, the designing and preparation of a specific labeled probe are very expensive. Additionally, it is costly to produce and use dideoxynucleotides.

Therefore, it is desirable even in the nucleotide sequencing of a specific region to directly determine a continuous sequence preferably with no use of the hybridization method. Further, desirably, the procedure can be carried out rapidly by a simpler method. Still further, compounds to be used for the reaction are preferably inexpensive and commonly used. Additionally, importantly, it is required to reduce a series of the cost by simplifying the assay apparatus and the reaction.

So as to overcome the problems, meanwhile, methods with no use of electrophoresis or hybridization are now proposed. These progress DNA extension reaction in a step-wise manner to characterize a nucleotide incorporated thereby. However, all of these progress DNA extension reaction one by one nucleotide. Therefore, a cycle should be repeated, which includes a step of once terminating extension reaction intentionally for the replacement or addition of the reaction solution and a step of detecting an inserted nucleotide. Thus, substantially, the process is more complicated.

International Publication WO 91/06678 describes a DNA method with no use of any gel as well as an apparatus for carrying out the method. The reaction requires 3'-blocked dTNP. Further, the method requires a process of separating the reaction solution from template DNA to separate the reaction solution in one reaction container from the template DNA during extension. Further, it is required to carry out the determination of inserted nucleotide using the reaction solution fractionated in the other reaction container. For continuous determination of such sequence, it is required to repeat the cycle. Therefore, the method is complicated in practical sense.

Furthermore, WO 94/00346 describes the nucleotide sequencing method with no need of electrophoresis. Even by the method, DNA extension reaction is essentially progressed in a step-wise manner. Additionally, the method inevitably involves a separation process of the reaction solution from template DNA, by procedures such as the immobilization of the template DNA.

### Disclosure of the Invention

It is considered that an ideal mode of continuous plural determination of nucleotide sequence is a method including directly assaying reactions continuously occurring in one reaction solution to enable the determination of DNA sequence on real time. However, it has been difficult to detect DNA sequence (alignment), which is supposedly positional information in a certain sense, based on reactions occurring in one reaction solution. Any of electrophoresis separation and step-wise DNA extension method has been considered as an essential process for avoiding the tough problems.

The invention overcomes the problems and provides a method capable of determining a nucleotide sequence of a single nucleotide or plural nucleotides by assaying reactions in one reaction solution.

In other words, the invention is described as follows.

### (Invention 1)

A method for nucleotide sequencing including determining the alignment in a sequentially extending polynucleotide by detecting a reaction product continuously generated in an extension reaction system following the extension reaction of polynucleotide chain.

### (Invention 2)

A method in the first aspect, the method including the extension reaction of polynucleotide chain..using DNA polymerase or RNA polymerase.

### (Invention 3)

A method in the first aspect, where the reaction product generated via the extension reaction of polynucleotide chain is a fluorescent substance.

### (Invention 4)

A method in the third aspect, where a nucleotide as a substrate for the extension reaction of polynucleotide chain is a fusion substance with a fluorescent substance and the reaction product generated via the extension reaction of polynucleotide chain is the fluorescent substance released from the fusion substance.

### (Invention 5)

A method in the fourth aspect, where the reaction to allow the release of the reaction product generated via the extension reaction of polynucleotide chain from the fusion substance is done with DNA polymerase or RNA polymerase.

### (Invention 6)

A method in the fourth aspect, where the fusion substance is deoxyribonucleotide 5'-triphosphate (dNTP) ester or ribonucleotide 5'-triphosphate (NTP) ester.

### (Invention 7)

A method in the third aspect, where the excitation and emission profile of the fluorescent substance varies in a manner dependent on each nucleotide as the substrate of the extension reaction.

### (Invention 8)

A method including comparing the gene sequences of plural biological species or different individuals on the basis of the nucleotide sequences determined using a method for nucleotide sequencing in the first to seventh aspects and thereby detecting the difference.

### (Invention 9)

A method for diagnosing the physiological feature of a biological species or an individual, using a method in the eighth aspect.

### (Invention 10)

A method for detecting a gene causing the difference in biological species or individuals, using a method in the eighth aspect.

### Best Mode for Carrying out the Invention

The extension reaction of polynucleotide chain allows the extension of polynucleotide chain by sequential bonding of one nucleotide complementary to a nucleotide in a polynucleotide sequence as template, in a manner dependent on the polynucleotide sequence. It is possible to synthetically prepare polynucleotide via chemical synthesis approach or enzymatic reaction. The reaction is carried out, preferably using DNA polymerase or RNA polymerase. These enzymes have esterase activity cleaving the ester bond in 3'-esterified nucleotide (I. Rasolonjatovo and S. R. Sarfati, Nucleosides & Nucleotides, 18, 1021-1022 (1999)). In case of using a fusion substance of a nucleotide bound with a fluorescent substance via ester bond as a substrate for the polynucleotide chain extension reaction, as described below, the ester bond is therefore cleaved following the extension reaction of the polynucleotide chain, so that the fluorescent substance is released and sequentially detected, to determine the alignment in the sequentially extending polynucleotide.

The term reaction product continuously generated in the extension reaction system following the extension reaction of the polynucleotide chain means a product generated during sequential bonding of one complementary nucleotide for the extension of polynucleotide chain, in a manner dependent on a template polynucleotide sequence. The reaction product may be any detectable, which is a chemical substance or a physical signal. The chemical substance may be an organic substance or an inorganic substance. Otherwise, radioisotopes can be used as such. The term continuous generation in the extension reaction system means continuous occurrence of the extension reaction of polynucleotide chain and the generation of the reaction product in one reaction system, but never includes step-wise extension reaction to alternately progress the detection and the extension reaction separately to detect the reaction product at each step. Additionally, the reaction product herein may be referred to the extending polynucleotide chain per se or a byproduct generated following the polynucleotide extension reaction. In view of ready detection described below, preferably, the byproduct generated following the polynucleotide extension reaction is the reaction product in accordance with the invention. A more preferable one of the reaction product is a fluorescent substance. By allowing a fluorescent substance with a different excitation and emission profile to be generated from each of the four nucleotides to be incorporated via the extension reaction, the excitation and emission profile is detected, to thereby detect the four fluorescent substances. By detecting the fluorescent substances, the individual nucleotides incorporated via the extension reaction can be detected. More preferably, nucleotides as the substrates of the extension reaction of polynucleotide chain are fusion substances with fluorescent substances, while the reaction product generated by the extension reaction of the polynucleotide chain is one of the fluorescent substances released from the fusion substances. More specifically, the fusion substance is deoxyribonucleotide 5'-triphosphate (dNTP) ester or ribonucleotide 5'-triphosphate (NTP) ester. As described above, the DNA polymerase or RNA polymerase to be used for the extension reaction has esterase activity cleaving the ester bond in 3'-esterified nucleotide. In case that a fusion substance of a nucleotide bound to a fluorescent substance via ester bond is used as a substrate of the extension reaction of polynucleotide chain, therefore, the ester bond is cleaved, following the extension reaction of polynucleotide chain, to release the fluorescent substance. By detecting the fluorescent substance, sequentially, the alignment in the sequentially extending polynucleotide can be determined.

The term detection of the reaction product means the detection of the presence of the reaction product in the extension reaction system or a reaction system partially separated from the reaction system, by a given detection method. The detection method varies, depending on the type of the reaction product. In case that different reaction products individually corresponding to A, T, G and C emerge, they are individually detected to determine the nucleotide types. In this case, such reaction products may sometimes be accumulated in the reaction system. In that case, the reaction products can be detected, sequentially, by examining the difference of the amount of a freshly generated reaction product from that of the already existing corresponding reaction product. Additionally, a method with no occurrence of the accumulation of any reaction product to be detected in the reaction system is more preferable. In case that the reaction product is to be a fluorescent substance, for example, a fluorescent substance with a different excitation and emission profile is fused with one of the four nucleotides as the substrates of the extension reaction. By subsequently detecting the excitation and emission profile using a fluorescent analyzer, the fluorescent substance can be detected. The detection of the fluorescent substance enables the detection of each nucleotide incorporated via the extension reaction.

The term determination of the alignment in the sequentially extending polynucleotide means the determination of nucleotide alignment in an objective polynucleotide, as the consequence of the detection of a reaction product generated via the extension reaction of polynucleotide in a manner dependent on the sequence of a polynucleotide chain, using the polynucleotide intended for the nucleotide sequencing as template. In case that a reaction product emitting fluorescence at a wavelength varying in a manner dependent on the type of the nucleotide for use in extension, the change of the fluorescent intensity at each wavelength over time is measured to determine the nucleotide alignment. In this case, plural reaction product molecules may simultaneously be generated in some case, depending on the plural template molecules. In case of simultaneous progress of the extension reactions of the plural molecules, the reactions are preferably synchronized among the molecules. Even when complete synchronization is impossible, a suitable computation procedure enables the calculation of the intended sequences. The computation procedure can be done via the analysis of the detected signals of such reaction products. Specifically, the computation can be done by averaging or inductive optimization for a speculative objective sequence.

The term comparison in gene sequence of plural biological species or different individuals to detect the difference therein means comparison of homologous genes among higher organisms of not only humans but also mouse, Drosophila, and nematodes and prokaryotic organisms such as microorganism to identify the characteristics of the sequences of the genes. For an identical species, the term also means comparison in the characteristics of the sequence of a specific gene among individuals to identify the difference.

The term diagnosis of the physiological feature of a biological species or an individual means the identification of the relation between physiological properties of a biological species or an individual and the nucleotide sequence thereof, to speculate the characteristic properties of the biological species or the individual on the basis of the gene sequences thereof for establishing diagnosis. Such diagnosis is applicable to the diagnosis of even human diseases. Specifically, nucleotide sequence is used for the examination of a gene type readily causing obesity or diabetes mellitus or for the diagnosis as to whether or not a specific pharmaceutical agent is therapeutically effective (Higuchi et al., Phermacogenetics 8, 87- (1998)). These are under way of research works and development, mainly as single nucleotide polymorphism analysis.

The term detection of gene causing the difference in biological species or individuals means to newly find single nucleotide polymorphism and the like. In this case, importantly, information about nucleotide sequence is available immediately at low cost. Additionally, a larger number of nucleotide sequence samples are more effective. Therefore, more rapid analysis of nucleotide sequence information at lower cost is very significant.

A specific mode for carrying out the invention is now described step by step. 1. Using a labeled dNTP (LdNTP) where the 3'-hydroxyl group of the deoxynucleotide is bound to a fluorescent substance via ester bond, and 2. progressing DNA extension reaction with DNA polymerase, 3. the incorporation of LdNTP and subsequent release of the fluorescent substance via the esterase activity of the DNA polymerase per se and next extension with the nucleotide LdNTP are progressed continuously in one reaction solution. Utilizing that the fluorescent substance can emit fluorescence only after the fluorescent substance is released to label individually A, G, T and C with fluorescent substances emitting different fluorescence, 4. the fluorescent substances continuously released following the extension reaction are sequentially detected, to obtain the nucleotide sequence information.

Each of the steps is now described in more detail. First, LdNTP can be prepared by the method described in the report of I. Rasolonjatovo and R.S. Sarfati, et al. (Nucleosides & Nucleotides, 16, 1757-1760 (1997) and Nucleosides & Nucleotides, 17, 2021-2025 (1998)). However, the type of fluorescent substance is not limited to those described therein. Additionally, any substance detectable is satisfactory, which is not necessarily fluorescent substance. As described below, further, the length of the fluorescent life of fluorescent substance can improve the efficiency of the nucleotide sequencing in accordance with the invention. Still additionally, the fluorescent substance is not necessarily bound via ester bond but is bound via any mode of bonding in such a fashion that the bond can be cleaved with DNA polymerase or RNA polymerase in a manner dependent on the bonding with template, to regenerate hydroxyl group.

Any commercially available DNA polymerase and RNA polymerase may be used satisfactorily for the extension reaction. The reaction solution is of a fundamental composition including template DNA containing a region for nucleotide sequencing, a primer for extension reaction, DNA polymerase and the like and appropriately including salts such as salts of Mg and the like and buffers for efficient reaction with LdNTP and DNA polymerase and the like. By elevating the rate of the extension reaction with DNA polymerase and the like, additionally, the rate of the nucleotide sequencing in accordance with the invention can be elevated. By reducing the rate of the extension, inversely, the sensitivity of the nucleotide sequencing can be elevated. The modification of the extension rate is satisfactorily carried out by a method for modifying the DNA polymerase per se and the like into variants or a method for modifying the conditions for the extension reaction. In case of the modification of DNA polymerase per se and the like, modification of not only the reaction rate but also the optimal range of the reaction temperature is also effective. In case of intending the modification of the reaction conditions, the modification can be done by adjusting the concentrations of substances to be added to the reaction solution, such as LdNTP, primer, template DNA and DNA polymerase or by adjusting the reaction temperature or pH.

LdNTP is incorporated in a manner depending on the sequence of template DNA. LdNTP of itself never emits fluorescence. However, fluorescence is emitted, when LdNTP is incorporated into the extending DNA in a manner dependent on the template and the ester bond is subsequently cleaved. By individually labeling A, G, T and C with fluorescent substances emitting different fluorescence types, then, a nucleotide type incorporated can be identified. The emergence of hydroxyl group at position 3' through the cleavage of the ester bond induces the insertion of a second LdNTP in a manner dependent on the template DNA sequence.

For fluorescent detection, the relation between the detection sensitivity and the time resolution is important. DNA extension reaction is a very rapid reaction under some conditions, but the reaction rate can be adjusted as described above. Principally, for example, the LdNTP concentration adjusted to a low concentration correspondingly lowers the reaction rate. For rapid nucleotide sequencing, it is suggested that extension reaction at a faster rate is preferable. Practically, however, the extension reaction should be set at a rate smaller than the time resolution of a detector when it is used for detecting fluorescence. Nonetheless, the time resolution of fluorescent analysis is generally as high as about 0.1 to 1.0 millisecond. Compared with the methods for extension reaction using gel or in a step-wise manner as described above, which require several seconds to several minutes on a single nucleotide basis, the time resolution is far higher. At a rate corresponding to the time resolution of a fluorescence analyzer, in other words, nucleotide sequence can be determined by a method permitting sequential fluorescence emission using LdNTP. The detection sensitivity of fluorescence is so high that even positional molecule can be detected. By increasing the amount of template DNA, further, the fluorescent intensity emitted can be elevated. It is then noted that in case of the existence of plural template DNA molecules, the polymerase extension reactions cannot perfectly be synchronized, so that emitted fluorescence is detected in the disorderly overlapped state. Even in this case, however, the original sequence can be determined by the computation process of the resulting data, as long as the plural template DNA molecules exist in a certain number. Practically, the optimal number of the DNA molecules to be extended and the optimal extension rate thereof are satisfactorily determined in view of the relation with the detection sensitivity and the reaction rate.

The method should be modified in that because fluorescent substances released via the esterase activity of DNA polymerase and the like accumulate in the reaction system, the fluorescent substances are also to be detected in an overlapped state with a fluorescent substance released from subsequent extension reaction. Therefore, the background for detection is increased as the extension reaction progresses. However, the problem can be overcome by deleting the background by the computation process of the counted value. For nucleotide sequencing for a long time, efficient processing of the background is effective. Further, the development of a process never permitting re-excitation of once emitted fluorescent substance, if it is possible, will be more satisfactory.

### Industrial Applicability

The invention enables nucleotide sequencing without fractionation procedure by electrophoresis or step-wise repetition procedure. By using the method, the time and cost required for nucleotide sequencing can be saved remarkably, leading to the highly efficient gene analysis. Consequently, pharmaceutical products can be developed efficiently, while the cost for gene diagnosis or gene therapy can be saved.

By using the method, the procedures for nucleotide sequencing can be done more rapidly in a more simplified manner. The applicability thereof in application fields is thereby improved more greatly, leading to the enlargement of the use thereof. To date, the technology for the analysis of human single nucleotide polymorphism is under way of development. However, even the technology cannot establish the comparison at the whole genome level. When the overall genome sequences of a great number of humans may possibly be determined for a short period of time, the comparison among individuals at the whole genome level will be possible, only when the processing speed of computer is improved. the simplification of the procedure for the determination of gene sequence enables gene diagnosis in a simpler manner, leading to the spread of gene diagnosis on clinical site more deeply.

## Claims

1. A method for nucleotide sequencing, including detecting a reaction product continuously generated in an extension reaction system following the extension reaction of poly-nucleotide chain and thereby determining the alignment in the sequentially extending polynucleotide.

2. The method according to claim 1, wherein DNA polymerase or RNA polymerase is used for the extension reaction of polynucleotide chain.

3. The method according to claim 1 or 2, wherein the reaction product generated following the extension reaction of poly-nucleotide chain is a fluorescent substance.

4. The method according to claim 3, wherein a nucleotide as a substrate of the extension reaction of polynucleotide chain is a fusion substance with a fluorescent substance and the reaction product generated following the extension reaction of polynucleotide chain is the fluorescent substance released from the fusion substance.

5. The method according to claim 4, wherein the reaction permitting the release of the reaction product generated following the extension reaction of polynucleotide chain from the fusion substance is done with DNA polymerase or RNA polymerase.

6. The method according to clam 4, wherein the fusion substance is deoxyribonucleotide 5'-triphosphate (dNTP) ester or ribonucleotide 5'-triphosphate (NTP) ester.

7. The method according to claim 3, where the excitation and emission profile of the fluorescent substance varies, depending on each nucleotide as the substrate of the extension reaction.

8. A method including comparing the gene sequences of plural biological species or different individuals on the basis of the nucleotide sequences determined using a method for nucleotide sequencing according to any one of claims 1 to 7 and thereby detecting the difference.

9. A method for diagnosing the physiological feature of a biological species or an individual, using a method according to claim 8.

10. A method for detecting a gene causing the difference in biological species or individuals, using a method according to claim 8.
